(19) Europäisches Patentamt
European Patent Office
Office européen des brevets

(11) Numéro de publication: **0 078 765 B1**

(12) # FASCICULE DE BREVET EUROPEEN

(45) Date de publication du fascicule du brevet: **26.02.86**

(51) Int. Cl.[4]: **C 07 C 103/50**, C 07 D 209/28, C 07 D 295/14, A 01 K 31/16, A 61 K 31/61, A 61 K 31/405

(21) Numéro de dépôt: **82810449.7**

(22) Date de dépôt: **26.10.82**

(54) **Dérivés de p-acylaminophénol à effet thérapeutique, procédé pour leur préparation, et composition à effet thérapeutique contenant lesdits dérivés à titre d'ingrédients pharmacologiquement actifs.**

(30) Priorité: **02.11.81 CH 7000/81**
**26.04.82 CH 2507/82**

(43) Date de publication de la demande:
**11.05.83 Bulletin 83/19**

(45) Mention de la délivrance du brevet:
**26.02.86 Bulletin 86/9**

(84) Etats contractants désignés:
**AT BE CH DE FR GB IT LI LU NL SE**

(56) Documents cités:
**BE - A - 587 453**
**DE - A - 2 625 195**
**FR - A - 2 392 975**

**CHEMICAL ABSTRACTS, vol.56, no.7, 2 avril 1962, colonne 7217gh, COLUMBUS, Ohio (US)**

**Le dossier contient des informations techniques présentées postérieurement au dépôt de la demande et ne figurant pas dans le présent fascicule.**

(73) Titulaire: **CERMOL S.A., CH-1902 Evionnaz (CH)**

(72) Inventeur: **Molnar, François, décédé (CH)**
Inventeur: **Szabo, Suzanne, Avenue de Sully, 1, CH-1814 La Tour-de-Peilz (CH)**
Inventeur: **Statkov, Peter, 6 rue de Roveray, CH-1207 Genève (CH)**
Inventeur: **Armijo, Manuel, Mateo Inurria 30, E-Madrid 16 (ES)**
Inventeur: **Sunkel, Carlos, Mateo Inurria 30, E-Madrid 16 (ES)**
Inventeur: **Cillero, Fernando, Mateo Inurria 30, E-Madrid 16 (ES)**

(74) Mandataire: **Vuille, Roman et al, c/o KIRKER & Cie S.A. 14, rue du Mont-Blanc Case Postale 872, CH-1211 Genève 1 (CH)**

EP 0 078 765 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1986

**Description**

L'invention a pour objet des composés nouveaux répondant à la formule

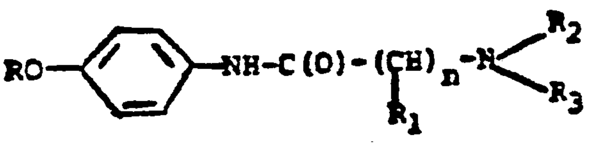

dans laquelle R représente un radical acryl dérivant d'un acide organique sélectionné parmi les acides

acétyl-salicylique, 1- (4-chlorobenzoyl) -5-méthoxy-2-méthyl-3-indolyl-acétique et 2- (4-isobutylphényl)propionique; n est égal à 0, 1,2 ou 3;

$R_1$ représente un atome d'hydrogène ou un groupe méthyle;

$R_2$ et $R_3$, qui peuvent être identiques ou differents, représentent chacun un groupe alkyle lineaire ou ramfilé, contenant de 1 à 4 atomes de carbone, 2-hydroxyethyl ou 2,3-dihydroxypropyl; ou dans laquelle le radical

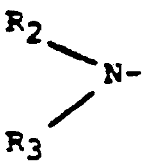

peut former un hétérocycle choisi parmi les morpholyle, pipéridyle, pyrrolidyle et leurs sels acides pharmaceutiquement acceptables.

L'invention a également pour objet un procédé pour la préparation desdits composés de formule I.

L'invention a finalement pour objet l'utilisation, à titre d'ingrédients pour la fabrication de compositions pharmaceutiques à effet analgésique et/ou anti-inflammatoire, de composés de formule

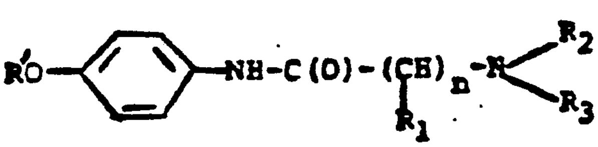

dans laquelle R' représente un atome d'hydrogène ou un radical acyle dérivant d'un acide organique tel que défini pour la formule I, l'indice n et les symboles $R_1$, $R_2$ et $R_3$ sont définis comme indiqué précédemment, et de leurs sels d'acides pharmaceutiquement acceptables.

Le N-acétyl-p-aminophénol a prouvé dans la pratique médicale une utilisation avantageuse par son activité analgésique. Toutefois, ce composé n' est pas dépourvu de certains effets toxiques aux plus fortes doses, notamment d'hépatotoxicité.

Il est également connu que certains dérivés du N-acétyl-paminophénol notamment des composés s'apparentant aux composés phénoliques de formule (I') définis ci-dessus présentent des proprietes thérapeutiques particulières. Selon DE-A-2625195, certains de ces composés sont utiles dans le traitement de maladies infectieuses, plus précisément de maladies dues à la présence d'agents infectieux du type Trypanosome ou Trichonomade. Selon BE-A-978453, d'autres dérivés phénoliques apparentes aux composés de formule (I') sont thérapeutiquement actifs comme antibilharziens.

Il a été trouvé, de façon surprenante, que certains composés résultant de la combinaison du p-aminophénol et d'acides aminés divers, plus précisément les composés de formule I, dans laquelle le symbole R représente un atome d'hydrogène, présentaient, aux doses thérapeutiques, des propriétés analgésiques intéressantes. Ils s'opposent à différents types de douleurs. En comparaison avec le produit de référence (N-acétyl-p-aminophénol) les représentants les plus actifs de ces nouveaux produits ont des qualités supérieures: ils sont moins toxiques et la différence entre les doses thérapeutiques et les doses provoquant des effets secondaires indésirables est supérieure: ils sont en outre bien solubles dans l'eau et peuvent être utilisés sous des formes galéniques injectables, ce qui n'est pas le cas pour le N-acétyl-p-aminophénol.

Il est bien connu, d'autre part, que certains acides organiques tels que l'acide acétylsalicylique, 5- (2,4-dif luorophényl) -acétylsalicylique, l'acide 1- (p-chlorobenzol)-5-méthoxyindole-acétique, certains acides arylpropioniques et certains acides anilino-phénylcarboxylique ou acétique sont utilisés dans la médecine humaine comme des antiinflammatoires et analgésiques non stéroidiens.

L'utilisation de tous ces acides cependant est généralement accompagnée de troubles gastro-intestinaux divers et plus particulièrement d'hémorragies et d'ulcères.

De façon surprenante, il a été trouvé que les composés de formule I' dans laquelle le symbole R' représente un radical acyle dérivant d'acides organiques tels que ceux déf inis plus haut sont des composés biologiquement très actifs et que, surtout, en comparaison avec les acides dont ils sont dérivés, les membres les plus intéressants de la série sont nettement moins toxiques, moins nocifs et moins ulcérigènes, tout en conservant une effilcacité remarquable et un index thérapeutique plus favorable.

Les composés de formule I' dans laquelle le symbole R représente un atome d'hydrogène (composées de formule IV) peuvent être prépares à partir des p-haloacylaminophénols correspondants de formule II. Les dits composés II sont décrits (Beilstein 13, 160), tandis que les amines secondaires de formule III sont des produits que l'on trouve dans le commerce; pour les essais suivants nous les avons utilisés en choisissant une qualité purum.

L'une des méthodes de preparation consiste à ajouter lentement le composé correspondant à la formule II dissous dans un solvant anhydre à la

solution de l'amine secondaire de formule III - à une température adéquate -, puis à filtrer les sels, évaporer le solvant et précipiter la base en ajoutant un solvant tel qu'acétate d'éthyle ou éther diisopropylique.

Avantageusement, on utilise aussi l'amine secondaire comme accepteur d'acide, c'est-à-dire dans 100-300 % d'excès. Dans les cas où le sel de l'amine secondaire est soluble dans le solvant tel qu'utilisé, on prend comme accepteur d'acide une amine tertiaire telle la triéthylamine ou la pyridine par exemple.

Une forme de réalisation particulièrement appréciée consiste à effectuer la dite réaction dans le diméthylformamide sec, ou la méthyléthylcétone anhydre.

On effectue favorablement la dite réaction entre les températures de 20-100°C. La durée de la réaction peut varier entre 2 heures et 5 jours.

Une forme de mise en oeuvre pour isolation du composé formé consiste à filtrer les sels, évaporer le solvant et l'excès d'amine sous vide, puis à précipiter la base en ajoutant un solvant approprié, tel qu'acétate d'éthyle ou éther diisopropylique.

Une autre forme d'isolation du composé désiré consiste à traiter le résidu d'évaporation dans un solvant comme isopropanol, acétate d'éthyle ou acétone avec un acide dissous dans un alcool, ou sous forme de gaz, de f açon à ce que le sel désiré précipite.

Les composés phénoliques ainsi obtenus peuvent être ensuite convertis en esters correspondants de formule I selon le procédé de l'invention, par réaction avec les halogénures des acides organiques mentionnés plus haut. Les chlorures de ces acides, en particulier, sont connus et facilement accessibles.

Une forme de mise en oeuvre du procédé de l'invention consiste à faire réagir un phénol de formule IV avec un chlorure d'acide correspondant en présence d'une base tertiaire dans un solvant approprié, à des températures adéquates, puis à laver le mélange réactionnel avec de l'eau jusqu'à neutralité, sécher et évaporer la phase organique, enfin à recristalliser le produit brut obtenu.

Selon l'invention, on utilise les composants de la réaction dans des quantités équimoléculaires, ou avec 10-15 % d'excès pour certains chlorures d'acides. Avantageusement on utilise une base tertiaire comme accepteur d'acide, telle que la triéthylamine ou la pyridine par exemple.

Selon une forme de mise en oeuvre particulièrement avantageuse, on fait réagir les composés dans une quantité appropriée d'un solvant organique, par exemple du chlorure de méthylène, du chloroforme ou du toluène sec. On effectue favorablement le dit procédé entre les températures de 10-110°C. La durée de la réaction peut varier entre 2 et 24 h.

Les nouveaux composés correspondant à la formule I forment des sels avec les substances acides. On peut obtenir ces sels en traitant le résidu d'évaporation dans un solvant comme isopropanol, acétone ou éther avec un acide dissous dans un alcool, ou sous forme de gaz, de façon à ce que le sel désiré précipite.

Une forme de réalisation particulièrement appréciée des dits sels consiste à effectuer la réaction entre un chlorure d'acide et le sel de dialkylaminoacétamidophénol de formule IV en présence d'une base tertiaire, en les utilisant dans des quantités sensiblement équimoléculaires. Ainsi on obtient directement le sel désiré, qu'on peut purifier par recristallisation.

Comme indiqué, certains des composés de formule I' présentent une activité analgésique alors que d'autres sont tout à la fois analgésiques et antiinflammatoires.

A titre de composés à effet analgésique, on peut citer entre autres les composés phénoliques de formule I' (R' = H) dans laquelle chacun des symboles R2 et R3 représente un radical alkyle inférieur: le p-N,N-diéthylglycylamidophénol et son chlorhydrate se sont montrés tout particulièrement efficaces. Des effets analgésiques significatifs ont été démontrés par l'emploi de doses comprises entre environ 100 et environ 300 mg/kg lors du test à l'acétylcholine chez la souris (administration par voie orale; ED environ 140 mg/kg).

A titre de composés à effet analgésique et antiinflammatoire, on peut citer par exemple le 1-(4-chloro-benzoyl)-5-méthoxy-2-méthyl-3-indolylacétate de 4-N,N-diéthylaminoacétamidophényle et son chlorhydrate. L'ef ficacité de ce dernier en tant qu' analgésique a été notamment démontrée par l'emploi de doses comprises entre env. 0,5 et env. 8 mg/kg lors du test à la phénylbenzoquinone chez la souris (ED50 env. 2,3 mg/kg).

Le dit chlorhydrate démontre en outre une activité antiinflammatoire significative lors du test d'inhibition de l'oedème plantaire du rat provoqué par la carraghénine ($ED_{50}$ env. 16,7 mg/kg). Ce composé présente également une activité antiarthritique et antipyrétique.

Pour obtenir l'effet thérapeutique désiré, on peut avantageusement utiliser les composés de formule I' en combinaison avec un excipient, un support ou un diluant inerte usuel.

Les exemples suivants illustrent la présnte invention sans aucunement en limiter la portée.

**Exemple 1**

A une solution de 77,5 g (1,06 mole) de diéthylamine dans 180 ml de diméthylformamide anhydre on ajoute goutte à goutte une solution de 49,3 g (0,265 mole) de N-chloroacétyl- p - aminophénol dans 80 ml de diméthylformamide anhydre. On effectue cette addition à une vitesse telle que la température du mélange ne dépasse pas 30°C; ensuite on chauffe - tout en agitant - pendant 2 heures à une température comprise entre 45 et 50°C, on laisse refroidir, on filtre le chlorhydrate du diéthylamine formé, puis on

évapore le solvant sous vide. En ajoutant au résidu (65,7 g) 450 ml d'acétate d' éthyle, une deuxième partie du chlorhydrate de diéthylamine précipite, on la filtre et on acidifie les eaux-meres avec de l'acide chlorhydrique dissous dans de l'isopropanol. On obtient ainsi 50,4 g du chlorhydrate de p-N,N-diéthylglycylamido-phénol fondant à 204-206° C. En évaporant des eaux-mères acides on peut récupérer 1 7,4 g du produit, qui donne après recristallisation dans 150 ml d' éthanol absolu 8,6 g du chlorhydrate; p. f. 204-206° C. Le rendement pour les deux fractions se situe à 86%.

**Exemple 2**

A la solution de 5,3 g (0,05 mole) de diéthanolamine et 7 ml (0,05 mole) de triéthylamine dans 20 ml de méthyléthylcétone anhydre on ajoute la solution de 9,3 g (0,05 mole) de N-α-chloroacéthyl-p-aminophénol dans 50 ml de méthyléthylcétone anhydre, puis on chauffe 10 heures à reflux. Apres refroidissement on filtre la triéthylamine chlorhydrate et on évapore la cétone sous vide. On reprend le résidu (18,7 g) dans 150 ml d'acétone, on le traite au charbon puis on acidifie avec une solution d'acide chlorhydrique dissous dans de l'isopropanol pour obtenir le chlorhydrate de p-N,N-di- (β-hydroxyéthyl) -glycylamidophénol; p.f. 141-142° C.

**Exemple 3**

En suivant les méthodes décrites dans les exemples 1 et 2 ci-dessus, on peut obtenir:

p-N,N-diméthylglycylamido-phénol. HC1. p.f. 227-229° C.

p-N,N-diisopropylglycylamido-phénol. HCl; p. f. 238-242° C.

p-N,N-diéthyl-α-alanylamido-phénol. HCl; p. f. 1 96-197° C.

p-N,N-dibutylglycylamido-phénol. HCl; p. f. 121-122° C.

1-N-morpholino-p-acétamido-phénol. HCl; p. f. 1 83-184° C.

p-N,N-dipropylglycylamido-phénol. HCl; p. f. 1 95-1 96° C.

p-N-éthyl-N-isopropylglycylamido-phénol. HCl; p. f. 204-205° C.

p-N- (β-hydroxyéthyl) -N-isopropylglycylamido-phénol.

HCl; p.f. 168-169° C.

p-N- (2, 3-dihydroxypropyl) -N-is opropylglycylamido-phénol maléate; p.f. 159-160° C.

1-N-piperidino-p-acétamido-phénol. HCl; p. f. 217-218° C.

**Exemple 4**

A une solution de 7,6 g (0,034 mole) de 4-N,N-diéthylaminoacétamidophénol, 4,05 g (0,04 mole) de triéthylamine dans 40 ml de toluène sec, on introduit 7,4 g (0,037 mole) de chlorure d'acide acétylsalicylique, de telle façon que la température du mélange ne dépasse pas 50° C. Une fois l'introduction terminée on chauffe 6 heures à ref lux. Après refroidissement on verse sur 50 ml de $H_20$, sépare les couches, lave la couche organique jusqu' à neutralité, seche sur $Na_2SO_4$ puis chasse le solvant sous vide. En recristallisant le résidu (1 3 g) dans 240 ml d'isopropyléther on obtient 9,1 g d'acétylsalicylate de 4-N,N-diéthylaminoacétami-dophényl; p. f. 99-100° C, avec un rendement de 69,6 %. Le chlorhydrate de cette base fond à 117-118° C.

Selon la méthode décrite ci-dessus on peut obtenir l'acétylsalicylate de 4-N,N-diméthylaminoacétamidophényl chlorhydrate; p.f. 152-157° C (déc.).

**Exemple 5**

A une solution de 56,4 g (0,15 mole) de chlorure de 1 - (4-chlorobenzoyl) -5-méthoxy-2-méthyl-3-indolylacétyle dans 500 ml de chloroforme sec et de 21 ml (0,15 mole) de triéthylamine on introduit par portion 38,8 g (0,15 mole) de 4-N,N-diéthylaminoacétamidophénol HCl. On agite le mélange pendant 6 heures à température ambiante, puis on lave avec un peu d'eau, sèche la solution chloroformique sur $Na_2SO_4$ et évapore le solvant à pression réduite. On recristallise le résidu dans de l'isopropanol pour obtenir, avec un rendement de 70 %, le chlorhydrate de 1- (4-chlorobenzoyl) -5-méthoxy-2-méthyl-3-indolylacétate de 4-N,N-diéthylaminoacétamido-phényle;p.f. 179-l8O° C (déc.).

**Exemple 6**

A une solution de 89,9 g (0,4 mole) de chlorure de 2- (p-isobutylphényl)-propionyle dans 300 ml de chlorure de méthylène sec, on ajoute 103,2 g de 4-N,N-diéthylamino-acétamidophénol HCl (0,4 mole), puis 56 ml (0,4 mole) de triéthylamine. On chauffe ce mélange pendant 2 heures à reflux, puis après refroidissement on lave à l'eau, sèche sur $Na_2SO_4$ anhydre puis évapore sous vide. Le résidu solide est recristallisé dans de l'isopropanol; on obtient ainsi le 2- (p-isobutylphényl) -propionate de 4-N,N-diéthylaminoacétamidophényle sous forme de chlorhydrate avec un rendement de 80 %; p.f. 153-155° C.

Selon la méthode décrite ci-dessus on peut obtenir le 2- (p-isobutylphényl) -propionate de 4-N,N-diisopropylaminoacétamidophényle chlorhydrate; p. f. 140-143° C (déc.).

## REVENDICATIONS

1 Composés répondant à la formule

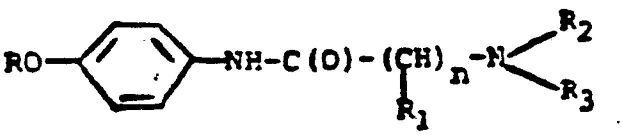

dans laquelle R représente un radical acyle dérivant d'un acide organique sélectionné parmi les acides acétyl-salicyliques, 1- (4-chlorobenzoyl) -5-méthoxy-2-méthyl-3-indolyl-acétique et 2- (4-isobutylphényl) -propionique;

n est égal à 0, 1, 2 ou 3;

$R_1$ représente un atome d'hydrogène ou un groupe méthyle.

$R_2$ at $R_3$, qui peuvent être identioues ou différents représentent chacun un groupe alkyle linéaire ou ramifié, contenant de 1 à 4 atomes de carbone, 2-hydroxyéthyl ou 2,3-dihydroxypropyl; ou dans laquelle le radical

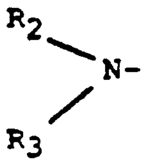

peut former un hétérocycle choisi purmi les morpholyle, pipéridyle, pyrrolidyle et leurs sels d'acides pharmaceutiquement acceptables.

2. Procédé pour la préparation d'un composé de formule (I) selon la revendication I dans laquelle R représente un radical acyle tel que défini à la revendication 1, caractérisé en ce qu'il consiste à faire réagir un chlorure d'acide correspondant, avec un phénol de formule

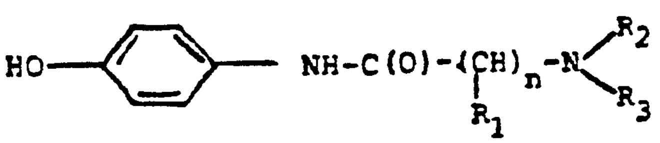

dans laquelle $R_1$, $R_2$ $R_3$ et n sont définis comme indiqué précédemment.

3. Procédé selon la revendication 2, caractérisé en ce qu' il consiste à faire réagir un phénol correspondant à la formule IV avec le chlorure d'acide en présence d'une base tertiaire, telle que la triéthylamime ou la pyridine.

4. Procédé selon les revendications 2 et 3, caractérisé en ce cu'il consiste à faire réagir les composés dans un solvant organique, de préférence dans le chloroforme, le chlorure de méthylène ou le toluène.

5. procédé selon l'ume des revendicatioms 2 à 4, caractérisé en ce qu' il consiste à opérer pendant la réaction à des températures comprises entre 10 et 110°C.

6. Procédé selon l'ume des revendications 2 à 5, caractérisé en ce qu'il consiste à laisser réagir les dits composés de 2 à 24 heures.

7. Procédé selon l'une des revendications 2 à 6, caractérisé en ce qu'il consiste à séparer le composé formé, à la fin de la réaction, en lavant à l'eau le mélange réactionnel, à évaporer le solvant et à recristalliser le produit brut.

8. Procédé selon l'une des revendicatios 2 à 7, caractérisé en ce qu'il consiste à faire réagir les bases ainsi obtenues dans une solution d'un solvant organique avec des acides minéraux ou organiques.

9. Procedé selon l'une des revendications 2 à 8, chractérisé en ce qu'il consiste à estérifier le chlorure d'acide avec un sel du phénol correspondant à la formule IV en présence d'une base tertiaire dans des quantités sensiblement équimoléculaires, pour obtenir directement le sel du composé de la formule I.

10. Utilisation de composés de formule

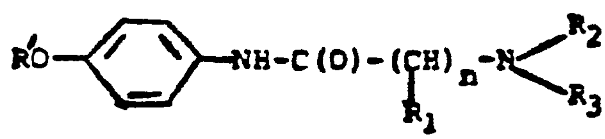

dans laquelle le symbole R' represente un atome d'hydrogène ou un radical acyle dérivant d'un acide organique tel que défini à la revendication 1, l'indice n et les symboles $R_1$, $R_2$ et $R_3$ sont définis comme à la revendication 1, et leurs sels d'acides pharmaceutiquement acceptables, à titre d'ingrédients pour la fabrication de compositions pharmaceutiques à effet analgésique et/ou anti-inflammatoire.

## Patentansprüche

1. Verbindungen der Formel

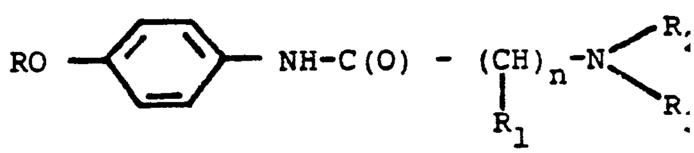

worin das Symbol R eine Acylgruppe darstellt, die aus einer organischen Säure, gewählt aus der Gruppe der Acetylsalicylsäure, 1- (4-Chlorobenzoyl) -5-methoxy-2-methyl-3-indolylessigsäure und 2- (4-Isobutylphenyl) -propionsäure, herkommt; n gleicht Null, 1, 2 oder 3;

$R_1$ ein Wasserstoffatom oder eine Methylgruppe darstellt;

$R_2$ und $R_3$, die gleich oder verschieden sein können, je eine lineare oder verzweigte Alkylgruppe, die von 1 bis 4 Kohlenstoffatome enthalten kann, eine 2-Hydroxyethylgruppe oder eine 2,3-Dihydroxypropylgruppe darstellen;

oder worin das Radikal

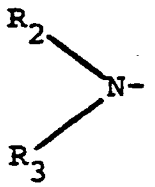

einen Heterozyklus darstellen kann, der aus der Morpholyl-, Piperidyl- oder Pyrrolidylgruppe gewählt ist, und deren pharmazeutisch vertragbare Salze mit Säuren.

2. Verfahren zur Herstellung von einer Verbindung der Formel I nach Anspruch 1, worin das Symbol R eine wie im Anspruch 1 definierte Acylgruppe darstellt, dadurch gekennzeichnet, dass man ein entsprechendes 8äurechlorid mit einer Phenolverbindung der Formel

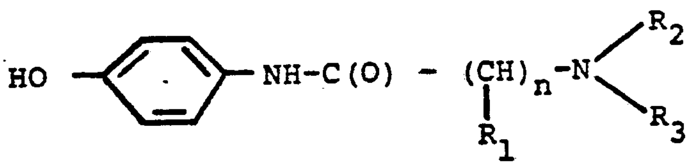

worin die Symbole $R_1$, $R_2$, $R_3$ und n wie oben definiert sind, reagieren lässt.

3. Verfahren nach Anspruch 2, dadurch gekennzeichnet, dass man ein Phenol der Formel IV in Gegenwart einer tertiären Base wie Triethylamin oder Pyridin mit dem entsprechenden Säurechlorid reagieren lässt.

4. Verfahren nach den Ansprüchen 2 und 3, dadurch gekennzeichnet, dass man die Verbindungen in Gegenwart eines organischen Lösungsmittels, vorzugsweise Chloroform, Methylenchlorid oder Toluen, reagieren lässt.

5. Verfahren nach einem der Ansprüche 2 bis 4, dadurch gekennzeichnet, dass die Reaktion bei einer Temperatur von 10 bis 110° C stattfindet.

6. Verfahren nach einem der Ansprüche 2 bis 5, dadurch gekennzeichnet, dass man die Verbindungen während einer Periode von 2 bis 24 Stunden reagieren lässt.

7. Verfahren nach einem der Ansprüche 2 bis 6, dadurch gekennzeichnet, dass man die gewünschte hergestellte Verbindung vor Ende der Reaktion trennt, dass man die Reaktionsmischung mit Wasser wäscht, dass man das Lösungsmittel abdämpft und dass man die rohe Verbindung auskristallisiert.

8. Verfahren nach einem der Ansprüche 2 bis 7, dadurch gekennzeichnet, dass man die so hergestellten Basen in einer Lösung eines organischen Lösungsmittels mit einer organischen oder mineralen Säure reagieren lässt.

9. Verfahren nach einem der Ansprüche 2 bis 8, dadurch gekennzeichnet, dass man das Säurechlorid mit einem Salz der Phenolverbindung der Formel IV umsetzt, in Gegenwart einer tertiären Base in ungefähr equimolaren Verhältnissen, um das Salz der Verbindung der Formel I direkt herzustellen.

10. Verwendung von Verbindungen der Formel

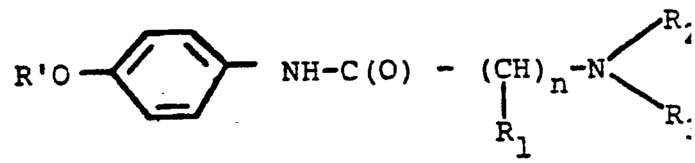

worin das Symbol R' ein Wasserstoffatom oder eine Acylgruppe darstellt, die aus einer wie im Anspruch 1 definierten organischen Säure herkommt, und worin die Symbole $R_1$, $R_2$ und $R_3$ und n wie im Anspruch I definiert sind, und deren pharmazeutisch vertragbare Salze mit Säuren als Zusatzstoffe zur Herstellung von Arzneimitteln mit einer schmerzstillenden und/oder Entzündung lindernden Wirkung.

**Claims**

A compound answering the formula

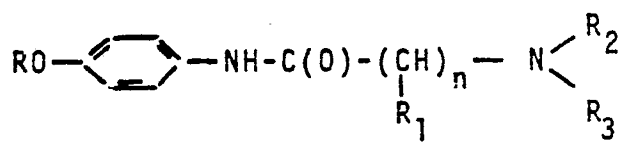

in which R stands for an acyl radical derived from an organic acid selected from the acetylsalicylic, 1-(4-chloro-benzoyl)-5-methoxy-2-methyl-3-indolyl acetic and 2-(4-isobutylphenyl)-propionic acids;

n is equal to 0,1 2 or 3;

$R_1$ stands for a hydrogen atom or a methyl group;

$R_2$ and $R_3$, which can be identical or different, each stand for a linear or branched alkyl group containing from 1 to 4 atoms of carbon, 2-hydroxyethyl or 2,3-hydroxypropyl; or in which the radical

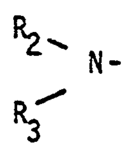

can form a heterocycle selected from morpholyl, piperydyl, pyrrolidyl and their salts of pharmaceutically acceptable acids.

2. A process for the preparation of a compound of formula (I) according to claim 1, in which R stands for a acyl radical such as defined in claim 1, characterized in that it consists in reacting a corresponding acid chloride with a phenol of the formula

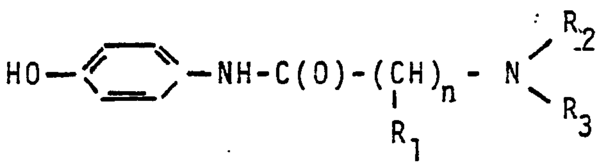

in which $R_1$,$R_2$, $R_3$ and n are defined as indicated previously.

3. A process according to claim 2, characterized in that it consists in reacting a phenol corresponding to the formula IV with the acid chloride in the presence of a tertiary base, such as triethylamine or pyridine.

4. A process according to claims 2 and 3, characterized in that it consists in reacting the compounds in an organic solvent, preferably in chloroform, methylene chloride or toluene.

5. A process according to one of claims 2 to 4, characterized in that it consists in operating during the reaction at temperatures compromised between 10 and 110°C.

6. A process according to one of claims 2 to 5, characterized in that it consists in letting said compounds react from 2 to 24 hours.

7. A process according to one of claims 2 to 6, characterized in that it consists:in separating the formed compound at the end of the reaction by washing the reaction mixture with water, evaporating the solvent, and recristallizing the raw product.

8. A process according to one of -claims 2 to 7, characterized in that it consists in reacting the bases thus obtained in a solution of an organic solvent with inorganic or organic acids.

9. A process according to one of claims 2 to 8, characterized in that it consists in esterifying the acid chloride with a salt of the phenol corresponding to the formula IV in the presence of a tertiary base in approximately equimolar quantities for obtaining directly the salt of the compound of formula I.

10. The utilization of compounds of the formula

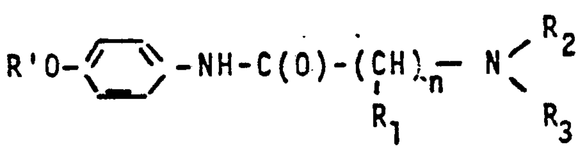

in which the symbol R' stands for a hydrogen atom or an acyl radical derived from an organic acid such as defined in claim 1 the index n and the symbols $R_1$, $R_2$, and $R_3$ are defined as in claim 1, and their salts of pharmaceutically acceptable acids as ingredients for the manufacture of pharmaceutical compositions with an analgesic and/or anti-inflammatory effects.